# EUROPÄISCHE PATENTSCHRIFT

(11) **EP 2 497 526 B1**
(45) Veröffentlichungstag und Bekanntmachung des Hinweises auf die Patenterteilung: **18.09.2013**
(21) Anmeldenummer: 12157035.2
(22) Anmeldetag: 27.02.2012
(51) Int. Cl.: A61N 1/05

(54) **Implantierbare Elektrodenleitung**
Implantable electrode lead
Ligne d'électrodes implantables

(30) Priorität: 08.03.2011 US 201161450144 P
(43) Veröffentlichungstag der Anmeldung: 12.09.2012
(73) Patentinhaber: BIOTRONIK SE & Co. KG, 12359 Berlin (DE)
(72) Erfinder: Dörr, Thomas, 12437 Berlin (DE); Kolberg, Gernot, 12161 Berlin (DE)
(74) Vertreter: Lindner-Vogt, Karin L.

(56) Entgegenhaltungen:
- WO-A1-2005/046470
- US-A- 4 913 147
- LENDLEIN A ET AL: "Biodegradable elasitc shape memory polymers for potential biomedical appplications", SCIENCE, AMERICAN ASSOCIATION FOR THE ADVANCEMENT OF SCIENCE, Bd. 296, Nr. 5573, 31. Mai 2002 (2002-05-31), Seiten 1673-1676, XP002514954, DOI: 10.1126/SCIENCE.1066102

## Beschreibung

Die Patentanmeldung betrifft eine implantierbare Elektrodenleitung wie beispielsweise Elektrodenleitungen für elektromedizinische Implantate wie implantierbare Herzschrittmacher, Kardioverter, Defibrillatoren oder Nerven- und Hirnstimulatoren.

Eine solche Elektrodenleitung besteht aus einem lang gestreckten Körper, der von außen aus einem isolierenden Material wie Silikon oder Polyurehtan besteht. Der Körper weist ein proximales und ein distales Ende auf. Am proximalen Ende befindet sich mindestens ein Stecker, welcher mit einem Anschluss im Anschlussgehäuse des Implantats - in der Regel eine Steckeraufnahme - verbunden werden kann. Der Stecker ist genormt und kann gemäß einer der Normen IS-1, IS-4 oder DF-1 gestaltet sein. Jede der elektrisch aktiven Kontakte des Steckers ist mit einer elektrischen Zuleitung elektrisch verbunden, welche wiederum an seinem distalen Ende in der Regel mit je einer elektrisch aktive Fläche (auch "Elektrodenpol" oder "Elektroden" genannt) elektrisch angebunden ist, welche am oder in der Nähe des distalen Endes liegt. Jede dieser Verbindungsleitungen ist isoliert geführt. Die elektrisch aktiven Flächen dienen dazu, an der zu behandelnden Körperpartie wie beispielsweise im oder am Herz, im oder am Gehirn oder an Nerven) eine elektrische Therapie zu induzieren oder aber Messsignale zur Diagnostik aufzunehmen.

Elektrodenleitungen weisen in der Regel mindestens einen vorgeformten und/oder versteiften Abschnitt des Elektrodenleitungskörpers auf. Diese Vorformungen oder Versteifungen dienen mehreren Zwecken. Einerseits können durch bestimmte Formen wie J-, Helix-, S-, oder Omegaformen die Befestigungseigenschaften der Elektrodenleitungen im Therapiegebiet wesentlich verbessert werden. Insbesondere bei Elektrodenleitungen, die im Coronarsinus liegen, sind aufgrund der Gefäßwandmorphologie keine anderen Befestigungsmöglichkeiten gegeben, als einen Abschnitt des Elektrodenleitungskörpers in diesem Gefäß zu verspannen. Die maximale laterale Erstreckung der Elektrodenleitung ist im vorgeformten und/oder versteiften Abschnitt auf den Durchmesser des als Einsatzort vorgesehenen Gefäßes vorbestimmt und beträgt für eine Koronarsinus-Elektrodenleitung etwa 8 mm. Weiterhin sind speziell für Coronarsinus-Elektrodenleitungen und Elektrodenleitungen, die beispielsweise eine Stimulation des hohen Septums ermöglichen sollen, Vorformungen eines Elektrodenleitungsabschnittes bekannt, mit Hilfe derer es vereinfacht wird, die Elektroden an bestimmten Stellen zu platzieren. Beispielsweise ist es in gewisser Weise kompliziert und erfordert viel Übung, eine Coronarsinus-Elektrodenleitung in den Coronarsinus einzuführen.

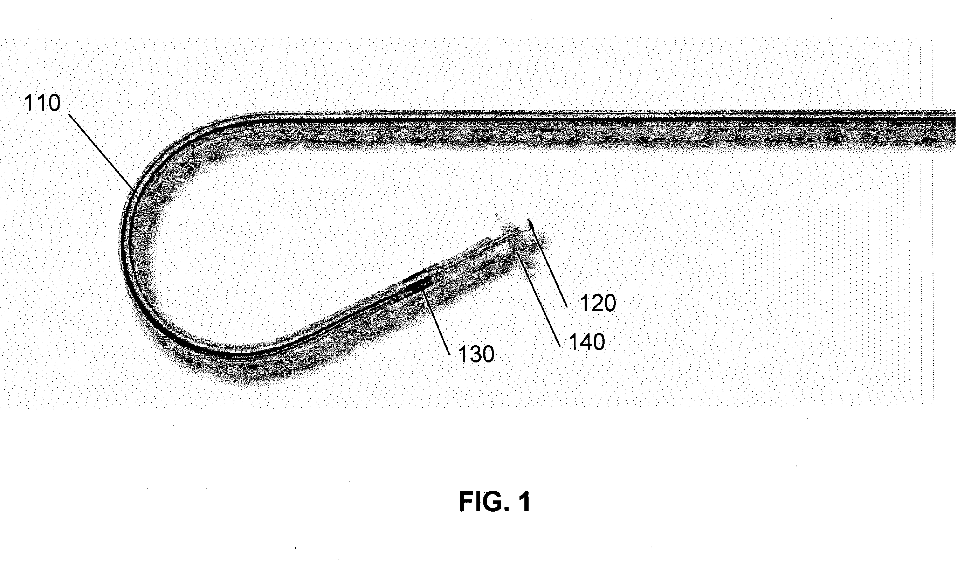

In FIG. 1 ist eine herkömmliche Elektrodenleitung mit J-Formung dargestellt. Diese ist in ihrem Aufbau durch einen Elektrodenkörper 110 gekennzeichnet, der in distalen Bereich J-förmig vorgeformt ist. Die Elektrode besitzt an ihrem proximalen Ende einen nicht dargestellten bipolaren Stecker nach IS-1 Norm und an ihrem distalen Ende zwei Elektrodenpole in Form eines Kopf- oder auch Tip-Elektrodenpols 120 und einen Ringelektrodenpol 130 sowie einen Mechanismus zur Elektrodenfixierung 140.

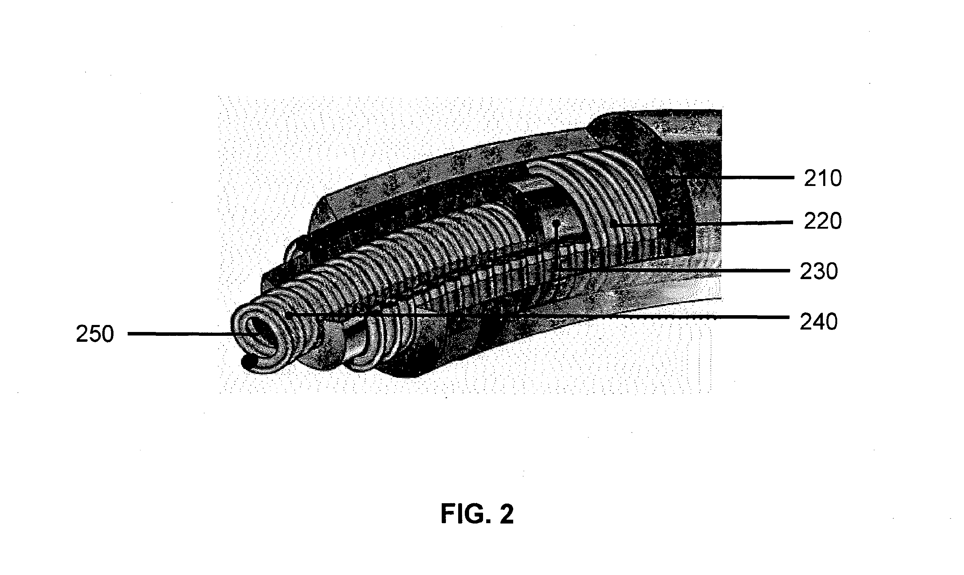

FIG. 2 zeigt den Aufbau der bekannten J-förmigen Elektrodenleitung Diese besitzt eine äußere Isolation 210, beispielsweise aus Silikon oder Polyurethan (PU). Eine äußere Zuleitungswendel 220 für den Anschluss der Ringelektrode 130, eine innere Isolation (230) und eine innere Zuleitungswendel 240 für den Anschluss der Tipelektrode 120. Da die J-Form im Elektrodenkörper vorgeformt ist, wird für die Implantation ein inneres Lumen 250 zur Aufnahme eines Führungsdrahtes benötigt, um die Elektrode bei der Einführung bis in den Vorhof gerade strecken und vor allem fixieren zu können.

Der vorgeformte und/oder versteifte Abschnitt einer solchen geformten Elektrodenleitung weist in der Regel eine elastisch vorgeformte und/oder versteifte Vorformungs- und Versteifungsstruktur beispielsweise in Form einer Seele aus getempertem Stahl MP35N auf. Die Elastizität der Leitung ist durch die geeignete Wahl der Stärke und der Materialbehandlungsbedingungen der Stahlseele derart vorbestimmt, dass sie durch den bei der Implantation eingesetzten Führungsdraht ohne weiteres gestreckt werden kann. Eine solche Seele ist aus der EP 0 951 920 A2 bekannt.

Diese bekannte Vorformung und/oder Versteifung der Elektrodenleitung hat mehrere Nachteile. Zum einen benötigen die Elektrodenleitungen aus dem Stand der Technik zur Implantation einen Führungsdraht, damit die Vorformung und/oder Versteifung ohne Verletzung der Gefäße an die Implantationsstelle verbracht werden können. Dazu wird ein lang gestreckter Führungsdraht in das soeben erwähnte innere Lumen geschoben und streckt somit den vorgeformten und/oder versteiften Abschnitt. Dieses Lumen bedeutet aber auch eine wesentliche Einschränkung der Konstruktionsfreiheit, da Elektrodenleitungen nur mit wesentlich größeren Durchmessern konstruiert werden können. Somit sind solche Elektrodenleitungen nicht mehr wahllos in allen Gefäßen einsetzbar.

Des Weiteren hat es sich nachteilig erwiesen, dass eine Elektrodenleitung im Bereich der vorgeformten und/oder versteiften Abschnitte eine gewisse Mindeststeifigkeit aufweisen muss, um ihrer Funktion als Verankerung oder Einführhilfe gerecht werden zu können. Diese relative Steifigkeit führt zu einer dauerhaften Belastung des Gewebes an der Fixationsstelle der Elektrodenleitung im Endokard oder in den Herzkranzgefäßen. Dadurch werden größere Reizschwellen verursacht und es kann ein höheres Risiko der Dislokation oder - wie in einigen Fällen dokumentiert - zu Perforationen des umgebenden Herzmuskels führen.

Des Weiteren sind schon Elektrodenleitungen bekannt, die sich aufgrund von bestimmten Werkstoffen während der Implantation verformen können. Aus der US 4,913,147 ist ein Beispiel für eine solche Elektrodenleitung bekannt, welche einen lang gestreckten vorgeformten Abschnitt mit einer Verformungs- oder Versteifungsstruktur aus Shape-memory Metall aufweist. Solche Elektrodenleitungen haben jedoch den Nachteil der mangelnden Flexibilität des einen lang gestreckten vorgeformten Abschnitts, was zu Gewebeschädigungen führen kann.

Daher besteht die Aufgabe der vorliegenden Patentanmeldung darin, die Nachteile der aus dem Stand der Technik bekannten Elektrodenleitungen zu beheben und eine Elektrodenleitung so zu verbessern, dass sie ohne Hilfsmittel eingeführt werden können und sie ohne schädigende Einwirkungen auf das umliegende Gewebe implantiert sein können.

Die Aufgabe wird durch den unabhängigen Anspruch 1 gelöst.

Gegenstand der Patentanmeldung ist eine Implantierbare Elektrodenleitung, welche einen lang gestreckten Elektrodenleitungskörper mit einem proximalen und einem distalen Ende, mindestens einer elektrischen Zuleitung, welche von einem elektrisch isolierenden Material umgeben ist, um die Zuleitungen gegen die Umgebung der Elektrodenleitung elektrisch zu isolieren, mindestens einer am oder in der Nähe des distalen Endes befindlichen und mit der mindestens einen elektrischen Zuleitung verbundenen elektrisch aktiven Fläche, durch die therapeutische Signale abgegeben und/oder diagnostische Signale aufgenommen werden können, mindestens einem am proximalen Ende befindlichem Stecker, welcher mit der mindestens einen elektrischen Zuleitung elektrisch verbunden ist und mit einem elektromedizinischen Implantat verbindbar ist, und mindestens einen vorgeformten und/oder versteiften Abschnitt des Elektrodenleitungskörpers, der eine zusätzliche Vorformungs- und Versteifungsstruktur aufweist, durch die der Abschnitt vorgeformt und/oder versteift ist, umfasst, wobei die zusätzliche Vorformungs- und Versteifungsstruktur ein Material umfasst, welches in einem ersten Zustand vorliegt, in dem es vorgeformt und/oder versteift ist, um die Vorformungs- und Versteifungsstruktur vorzuformen und/oder zu versteifen, und welches durch Energieeintrag und/oder Degradation in einen zweiten Zustand überführbar ist, in welchem die Form und/oder die Steifigkeit des Materials und damit der Vorformungs- und Versteifungsstruktur gegenüber dem ersten Zustand verändert ist.

Der Gegenstand zeichnet sich dadurch aus, dass die Vorformungs- und Versteifungsstruktur einen gitterartigen Aufbau aufweist und den kompletten Elektrodenleitungskörper umschließt.

Dadurch wird sichergestellt, dass die Elektrodenleitung in einem ersten Zustand die benötigte Mindeststeifigkeit aufweist, die benötigt wird, um die Elektrodenleitung ohne den auch versteifenden Effekt eines Führungsdrahtes einzuführen. Gleichzeitig wird sichergestellt, dass nach einer gewissen Zeit, wenn diese Mindeststeifigkeit nicht mehr benötigt wird, die Flexibilität so erhöht wird, dass einerseits die Vorformung und andererseits auch die Versteifung nahezu komplett aufgegeben ist. Diese Verformung und/oder Versteifung wird auch deswegen nicht mehr benötigt, weil der betreffende Abschnitt in das Gewebe eingewachsen ist.

Zudem ist es möglich, bei dieser Ausgestaltung des vorgeformten und/oder versteiften Abschnittes auf ein Lumen zur Aufnahme eines Führungsdrahtes oder eines Mandrins komplett zu verzichten, da diese Funktion durch die anfängliche Versteifung und Vorformung übernommen wird.

Ideal hat es sich herausgestellt, wenn die Vorformungs- und Versteifungsstruktur aus einem Shape Memory Polymer (SMP) und/oder einem biodegradierbares Polymer besteht. Diese beiden Materialien sind hervorragend geeignet, die geforderte Zustandänderung abzubilden. Biodegradierbare Polymere verlieren ihre Steifigkeit durch Degradation. Besonders geeignet hierfür sind die biodegradierbaren Polymere Polydioxanon, Polyglycolid, Polycaprolacton, Polylactide (Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(1-Lactid-co-trimethylen carbonat, Triblockcopolymere), Polysaccharide (Chitosan, Levan, Hyaluronsäure, Heparin, Dextran, Cellulose etc.), Polyhydroxyvalerat, Ethylvinylacetat, Polyethylenoxid, Polyphosphorylcholin, Fibrin, Albumin.

SMPs verlieren unter Erwärmung über eine Übergangstemperatur ihre Form. Die Form kann dabei herstellungsbedingt vorgeformt werden, so dass sich die Vorformung nach Übersteigen der Übergangstemperatur einstellt. Als Beispiel für ein SMP sind Poly(styrene-Block-Butadiene), Polyetherketone (PEEK) oder Polymethylmetakrylate (PMMA) genannt.

Ebenso einsetzbar sind auch Butylacrylate, deren Seitenketten über Zimtsäuregruppen unter der Einwirkung von UV-Licht vernetzt werden und so eine erste Geometrie ausprägen. Durch Einwirkung von Licht anderer Wellenlänge über einen entsprechenden Lichtleiter können diese Vernetzungen aufgelöst werden und so eine zweite Geometrie eingestellt werden.

Besonders vorteilhaft sind diese beiden Eigenschaften kombiniert, indem das soeben genannte Shape Memory Polymer biodegradierbar ist. Als beispielhafte Materialien sind Polyalkanoate, Polyhydroxylsäureverbindungen, Polyphosphazene, Polyetherester oder Polyanhydride zu nennen.

Gemäß der bevorzugten Ausführung weist die Vorformungs- und Versteifungsstruktur erste Verstrebungen mit einem ersten Wickelsinn und zweite Verstrebungen mit einem zweiten Wickelsinn auf, wobei der erste und zweite Wickelsinn in verschiedener Richtung um den Elektrodenleitungskörper herum angeordnet sind, so dass sich die beiden Verstrebungen an Kreuzungspunkten kreuzen. Dabei sind die ersten und zweiten Verstrebungen lang gestreckt und weisen in sich keine Winkel auf.

Dabei sind die ersten und zweiten Verstrebungen an den Kreuzungspunkten miteinander verbunden, insbesondere verklebt oder verschweißt, und die Kreuzungspunkte zusätzlich durch Querstreben miteinander verbunden. Die ersten und zweiten Verstrebungen haben keine gespeicherte Form haben und die Querstreben sind verstreckt.

Das Material der Vorformungs- und Versteifungsstruktur ist ausgebildet, mindestens zwei Zustände anzunehmen. Dabei ist die Vorformungs- und Versteifungsstruktur im ersten Zustand des Materials so ausgebildet, dass der vorgeformte Abschnitt der Elektrodenleitung eine versteifte J-, eine versteifte Helix-, eine versteifte S-, eine versteifte Omega- oder eine versteifte gerade lang gestreckte Form aufweist. Dadurch kann das Einführen einer Elektrodenleitung und das temporäre Platzieren im Therapiegebiet bis zum Einwachsen der Elektrodenleitung sichergestellt werden, da die notwendige Mindeststeifigkeit vorliegt, die die Fixierung ermöglich. Im zweiten Zustand muss der vorgeformte Abschnitt wesentlich flexibler sein und seine Vorformung und Versteifung nahezu komplett verlieren. Deshalb ist die Vorformung der Vorformungs- und Versteifungsstruktur im zweiten Zustand des Materials so ausgebildet, dass der vorgeformte Abschnitt der Elektrodenleitung eine im Vergleich zum ersten Zustand formlose und elastische Form aufweist, insbesondere lang gestreckt und flexibel ist. Dadurch wird erreicht, dass der Druck auf das umliegende Gewebe minimiert ist und keine störenden Einflüsse mehr durch die vorgeformte und versteifte Elektrodenleitung erfährt.

Besonders vorteilhaft ist es, wenn die Elektrodenleitung im vorgeformten und/oder versteiften Abschnitt mehrere Zustände annehmen kann, die an die verschiedenen Phasen einer Implantation angepasst sind. So ist es vorteilhaft, wenn sich der genannte Abschnitt in einer ersten Phase als lang gestreckt und steif ausbildet, um so das Führen durch die Gefäße beispielsweise zum Herzen sicherzustellen, in einer zweiten Phase einen vorgeformten und versteiften Zustand annimmt, in dem die Elektrodenleitung entweder eine Führung-beispielsweise eine Vorbiegung, um leichter in den Coronarsinus einzudringen- oder eine Fixierung annimmt, mit Hilfe derer die Elektrode befestigt wird. Abschließend kann der Abschnitt die Form verlieren und flexibel werden, wenn die Elektrode an der gewünschten Stelle festgewachsen ist. Deswegen ist das Material der Vorformungs- und/oder Versteifungsstruktur gegebenenfalls durch Energieeintrag und/oder Degradation zusätzlich in einen Zwischenzustand überführbar, der sich zwischen dem ersten und zweiten Zustand befindet, in dem sie vorgeformt und/oder versteift ist, um die Vorformungs- und Versteifungsstruktur in einer vom ersten Zustand unterschiedlichen Art und Weise vorzuformen und/oder zu versteifen. Vorzugsweise weist die Vorformungs- und Versteifungsstruktur im ersten Zustand des Materials eine versteifte lang gestreckte Form, im Zwischenzustand des Materials eine versteifte J-, eine versteifte Helix-, eine versteifte S- oder eine versteifte Omega-Form, und im zweiten Zustand des Materials eine im Vergleich zum ersten oder Zwischenzustand formlose und elastische Form auf. Insbesondere ist sie lang gestreckt und flexibel ist.

Wie schon beschrieben, ist das Material der Vorformungs- und Versteifungsstruktur aus einem Material, das durch Energieeintrag oder Degradation seinen Zustand ändert. Dabei wird insbesondere die Zuführung der Energie in Form von Körperwärme erfolgen. Im normalen Zustand weist der menschliche Körper eine Temperatur im Durchschnitt von 36,5° Celsius auf. Neben der Formänderung soll jedoch auch eine Flexibilisierung erfolgen, welche durch die Degradation des Materials erfolgt. Deshalb stellt sich der Zwischenzustand des Materials durch Energieeintrag, insbesondere Wärme im Bereich von 35°C bis 40°C, und/oder Degradation, und der zweite Zustand nur durch Degradation ein. Die Degradation kann dabei dermaßen erfolgen, dass zuerst eine schnelle Degradation erfolgt, beispielsweise in einem mehrschichtigen Aufbau des Materials durch eine obere schnell degradierbare außen liegende erste Polymerschicht und ein zweites Polymer, welches vollständig von der ersten Polymerschicht umschlossen ist und welches langsam degradiert. Das zweite Polymer kann dabei beispielsweise ein biodegradierbares SMP sein.

Um den verschiedenen genannten Phasen zeitlich gerecht zu werden, stellen sich die verschiedenen genannten Zustände in unterschiedlichen Zeithorizonten ein. So stellt sich der Zwischenzustand des Materials innerhalb von 0,5 bis 10 min und der zweite Zustand innerhalb von 1 bis 140 Tagen ein.

Es zeigen:
- FIG. 1: eine Elektrodenleitung mit einem J-förmig vorgeformten Abschnitt aus dem Stand der Technik
- FIG. 2: den koradialen Aufbau einer Elektrodenleitung aus dem Stand der Technik
- FIG. 3A und FIG. 3B: einen vorgeformten Abschnitt einer Elektrodenleitung des Gegenstandes der Patentanmeldung
- FIG 4A bis FIG. 4D: einen beispielartigen Aufbau einer gitterartigen Verformungs- und Versteifungsstruktur aus Shape Memory Polymer (SMP)
- FIG. 5A und FIG. 5B: die beispielhafte Anordnung der Verformungs- und Versteifungsstruktur im vorgeformten Abschnitt

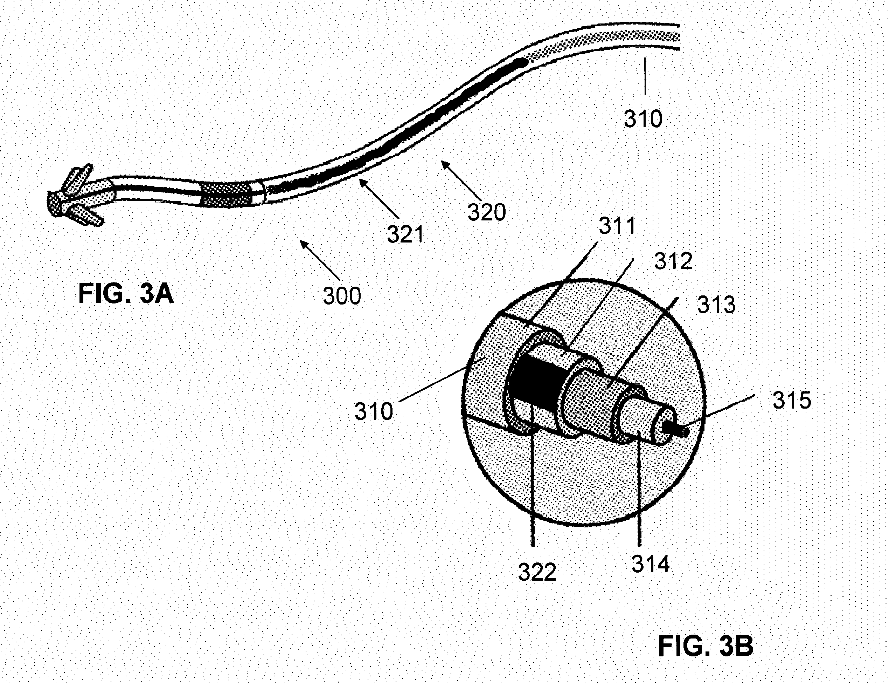

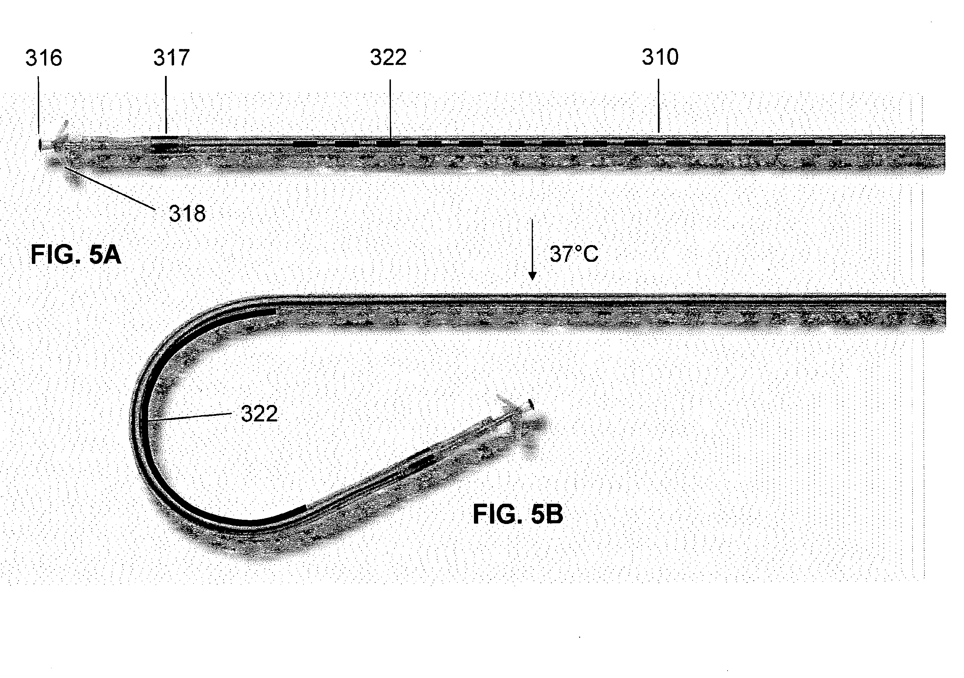

Die in den Figuren 3A, 3B, 5A, 5B gezeigten Ausführungsbeispiele sind nicht Teil der Erfindung.

In der FIG 3A und FIG. 3B ist ein vorgeformter und versteifter Abschnitt einer Elektrodenleitung 300 des Gegenstandes der Patentanmeldung gezeigt, der den Aufbau einer SMP-J-Elektrodenleitung dargestellt. Der Elektrodenleitungskörper 310 weist einen vorgeformten Abschnitt 320 auf, der mit einer Vorformungs- und Versteifungsstruktur 321 versehen ist. Einerseits kann diese Vorformungs- und Versteifungsstruktur 321 den kompletten Elektrodenleitungskörper umschließen.

Andererseits kann, wie in der vergrößerten Teilschnittdarstellung der FIG. 3B dargestellt, die Vorformungs- und Versteifungsstruktur 321 ein einseitig im vorgeformten und versteiften Abschnitt 320 sein, der bevorzugt im distalen Bereich der Elektrodenleitung 300 liegt. Dabei kann es sich um einen zunächst unter Temperatureinfluss verformten SMP-Streifen 322 handeln. Der Elektrodenleitungskörper 310 besteht hier aus einer äußeren, biodegradierbaren Gleit- und Schutzschicht 311. Diese Gleit- und Schutzschicht 311 schützt den darunter liegenden, mit einem Haftvermittler auf die äußere Isolation 312 aufgeklebten SMP-Streifen 322.

Der Innenaufbau der Elektrodenleitung 300 innerhalb der äußeren Isolation 312 ähnelt dem Aufbau einer herkömmlichen Elektrodenleitung mit dem Unterschied, dass kein Führungsdrahtlumen vorgesehen ist, da die Funktion des Führungsdrahtes durch die Vorformungs- und Versteifungsstruktur erfüllt wird. Sie umfasst also eine äußere Elektrodenwendel 313, die durch die innere Isolation 314 vom inneren Leiter 315 abgegrenzt wird.

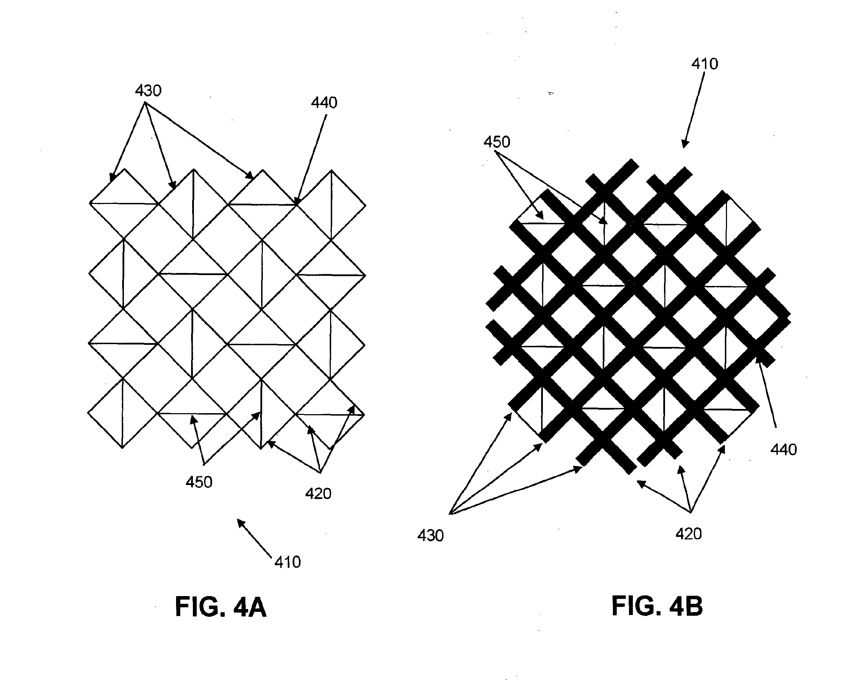

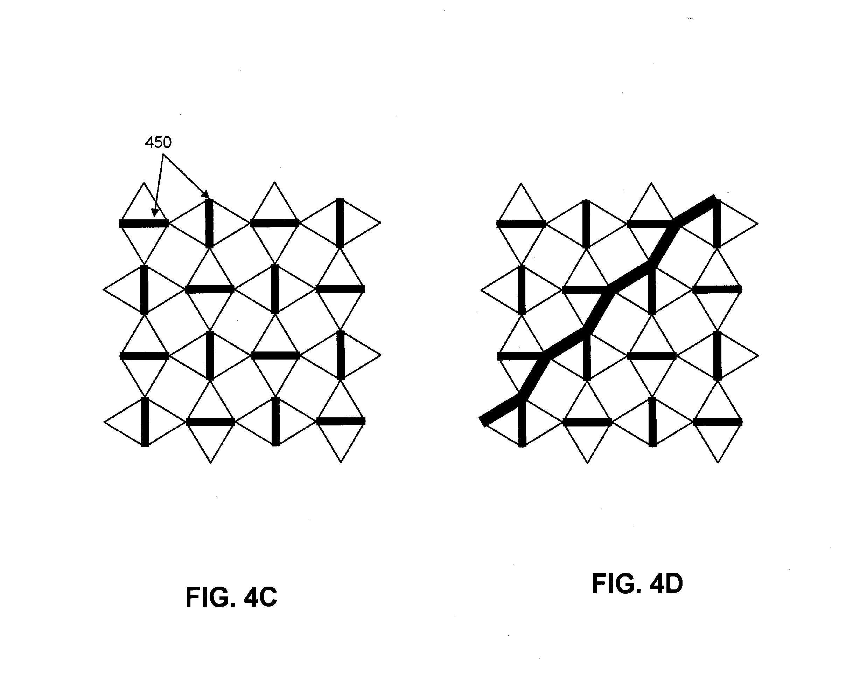

FIG. 4A bis FIG. 4D zeigen einen beispielartigen Aufbau einer gitterartigen Vorformungs- und Versteifungsstruktur aus SMP, die nicht wie in FIG. 3B einseitig als Streifen angebracht ist, sondern wie schon erwähnt den kompletten Elektrodenleitungskörper umschließen kann. Eine gitterartige Struktur 410 aus einem SMP-Netz ist um die Elektrode gewunden. Die Struktur weist erste Verstrebungen 420 mit einem ersten Wickelsinn und zweite Verstrebungen 430 mit einem zweiten Wickelsinn auf, wobei der erste und zweite Wickelsinn in verschiedener Richtung um den Elektrodenleitungskörper herum angeordnet sind, so dass sich die beiden Verstrebungen an Kreuzungspunkten 440 vorzugsweise in einem nahezu rechten Winkel kreuzen. Die ersten und zweiten Verstrebungen sind dabei lang gestreckt und weisen in sich keine Winkel auf. Die ersten und zweiten Verstrebungen sind an den Kreuzungspunkten 440 miteinander verbunden, beispielsweise verklebt oder verschweißt, so dass die beiden Verstrebungen 420 und 430 nicht gegeneinander verschiebbar sind. Auf diese Weise wird die Elektrode versteift. Bei einer Biegung der Elektrode nehmen die ersten und zweiten Verstrebungen 420 und 430 Zugkräfte auf und erhöhen die für die Biegung erforderliche Kraft. Die Kreuzungspunkte 440 sind in der dargestellten Weise zusätzlich durch Netzelemente - in diesem Beispiel durch Querstreben 450 - miteinander verbunden. Während die ersten und zweiten Verstrebungen 420 und 430 keine gespeicherte Form haben, sind die Querstreben 450 verstreckt. Werden sie während der Implantation der Körperwärme des Patienten ausgesetzt und verkürzen sie sich nach ca. 10 Minuten (FIG. 4C und FIG. 4D).

Das hat zur Folge, dass die Gitterstruktur verzerrt wird. Die ersten und zweiten Verstrebungen 420 und 430 sind nicht mehr gerade und wirken Zugkräften elastisch entgegen. Diese Elektrodenleitung ist am vorgeformten Abschnitt während der Implantation steif und lässt sich vom Anwender leicht vorschieben und dirigieren. Nachdem die Elektrodenleitung am optimalen Punkt in einem Gefäß wie beispielsweise dem Herzen fixiert wurde, wird sie weich und elastisch. Sie stellt dann für das Gefäßsystem keinen mechanischen Widerstand mehr dar und ist dauerhaft stabil.

In einem weiteren Ausführungsbeispiel wird der Shape Memory Effekt in anderer Richtung ausgenutzt. Während sich im soeben genannten Beispiel die ersten Verstrebungen 420 einer Gitterstruktur verlängern, kann das Gitter auch so ausgelegt sein, dass sich anstatt der ersten Verstrebungen 420 die zweiten Verstrebungen 430 verlängern und sich so die statischen Eigenschaften des Gitters ändern.

In einem weiteren Ausführungsbeispiel wird der Shape Memory Effekt in beiden Richtungen ausgenutzt, so dass sich in bestimmten Abschnitten der Verformungs- und Versteifungsstruktur die ersten Verstrebungen 420 verlängern und in anderen Abschnitten die zweiten Verstrebungen 430. Einzelne Verstrebungen 420 oder 430 verlängern sich nach Einführung in den Patienten, andere verkürzen sich. Auf diese Weise wird die Gitterstruktur aufgeweicht.

In einem weiteren Ausführungsbeispiel - dargestellt anhand einer Elektrodenleitung für das hohe Septum - wird die Gitterstruktur der Vorformungs- und Versteifungsstruktur durch Ausnutzung des Shape Memory Effektes und der Degradation des Materials so verändert, dass die Elektrodenleitung im Bereich des vorgeformten Abschnittes außerhalb des Körpers und während des Vorschiebens versteift ist. In das Gefäßsystem eingeführt, nimmt der Elektrodenleitungskörper über die Phasen der Implantation unterschiedliche Formen an, die zeitlich auf die Implantation und dem Einwachsverhalten abgestimmt sind.

Zu Beginn ist die Elektrodeleitung im Bereich des vorgeformten Abschnittes gerade und sehr steif. Der Elektrodenleitungskörper kann so sehr leicht in das Einführbesteck eingeführt und vorgeschoben werden.

Nach ca. 10 Sekunden verkürzen sich langsam die Netzelemente der Vorformungs- und Versteifungsstruktur im Bereich des vorgeformten Abschnittes, der vorzugsweise an der Elektrodenspitze liegt, und stören so die starre Gitterstruktur. Der vorgeformte Abschnitt wird in diesem Bereich weicher. So kann die Elektrodenleitung leicht und ohne das Gewebe zu traumatisieren in den rechten Ventrikel des Herzens geschoben werden.

Nach ca. 1 Minute haben sich die Netzelemente der Verformungs- und Versteifungsstruktur auf einer Seite des Umfangs so weit verkürzt, dass die Elektrodenleitung beispielsweise einen großen Bogen ausprägt. Die Elektrodenleitung bewegt sich so selbständig in die Richtung des Ausflusstraktes des rechten Ventrikels. Nach einer weiteren Minute verkürzen sich wenige Elemente am vorgeformten Abschnitt etwa auf der gegenüberliegenden Seite des Bogens und formen einen kleinen entgegen gerichteten Bogen aus. Die Elektrodenspitze zeigt nun auf das Hisbündel des Patienten und kann mit geeigneten Haltemechanismen im Gewebe fixiert werden. Innerhalb weniger Wochen degradiert die Vorformungs- und Versteifungsstruktur aus degradierbarem Shape Memory Material. Die Elektrodenleitung ist bis dahin eingewachsen. Sie wird nun weich und hoch elastisch für einen atraumatischen Langzeitbetrieb.

In FIG 5A und 5B ist eine beispielhafte Anordnung der Verformungs- und Versteifungsstruktur im vorgeformten Abschnitt gezeigt. Die Shape-Memory J-Elektrodenleitung wird in FIG. 5A im Auslieferungszustand gezeigt. Der gerade Elektrodenleitungskörper 310 besitzt elektrisch leitende Flächen in Form einer Tip- und einer Ringelektrode 316 und 317 sowie einen Fixierungsmechanismus 318. Im Bereich der gewünschten J-Verformung befindet sich die Vorformungs- und Versteifungsstruktur, ausgebildet als Shape-Memory Polymer Streifen 322 in gestreckter Form.

Wird diese Elektrodenleitung nun implantiert, dann erwärmt sich der Shape-Memory Streifen 322 und verkürzt sich auf seine eigentliche Länge, so dass dabei die typische J-Form innerhalb von 0,5 bis 15 Minuten ausgeprägt wird.

In der Ausführung dieser Elektrodenleitung mit biodegradierbarer Schutzschicht und biodegradierbarem SMP-Streifen kann nach Einwachsen der Elektrodenleitung die mechanische Spannung im Bereich des vorgeformten J-Abschnittes deutlich reduziert werden und so eine Entlastung des Herzgewebes im Bereich der Elektrodenfixierung erreicht werden.

## Patentansprüche

1. Implantierbare Elektrodenleitung, umfassend:
- einen lang gestreckten Elektrodenleitungskörper mit einem proximalen und einem distalen Ende, mindestens einer elektrischen Zuleitung, welche von einem elektrisch isolierenden Material umgeben ist, um die Zuleitungen gegen die Umgebung der Elektrodenleitung elektrisch zu isolieren,
- mindestens einer am oder in der Nähe des distalen Endes befindlichen und mit der mindestens einen elektrischen Zuleitung verbundenen elektrisch aktiven Fläche, durch die therapeutische Signale abgegeben und/oder diagnostische Signale aufgenommen werden können,
- mindestens einem am proximalen Ende befindlichem Stecker, welcher mit der mindestens einen elektrischen Zuleitung elektrisch verbunden ist und mit einem elektromedizinischen Implantat verbindbar ist, und
- mindestens einen vorgeformten und/oder versteiften Abschnitt des Elektrodenleitungskörpers, der eine zusätzliche Vorformungs- und Versteifungsstruktur (410) aufweist, durch die der Abschnitt vorgeformt und/oder versteift ist, wobei die zusätzliche Vorformungs- und Versteifungsstruktur ein Material umfasst, welches in einem ersten Zustand vorliegt, in dem es vorgeformt und/oder versteift ist, und welches durch Energieeintrag und/oder Degradation in einen zweiten Zustand überführbar ist, in welchem die Form und/oder die Steifigkeit des Materials und damit der Vorformungs- und Versteifungsstruktur gegenüber dem ersten Zustand verändert ist,
**dadurch gekennzeichnet, dass**
die Vorformungs- und Versteifungsstruktur (410) einen gitterartigen Aufbau aufweist und den kompletten Elektrodenleitungskörper umschließt.

2. Implantierbare Vorrichtung nach Anspruch 1, **dadurch gekennzeichnet, dass** die Vorformungs- und Versteifungsstruktur (410) erste Verstrebungen (420) mit einem ersten Wickelsinn und zweite Verstrebungen (430) mit einem zweiten Wickelsinn aufweisen, wobei der erste und zweite Wickelsinn in verschiedener Richtung um den Elektrodenleitungskörper herum angeordnet ist, so dass sich die beiden Verstrebungen an Kreuzungspunkten (440) kreuze, wobei
die ersten und zweiten Verstrebungen (420, 430) lang gestreckt sind und in sich keine Winkel aufweisen,
die ersten und zweiten Verstrebungen (420, 430) an den Kreuzungspunkten (440) miteinander verbunden, bevorzugt verklebt oder verschweißt sind die beiden Verstrebungen 420 und 430 nicht gegeneinander verschiebbar sind,
die Kreuzungspunkte (440) zusätzlich durch durch Querstreben (450) miteinander verbunden sind, und
die ersten und zweiten Verstrebungen (420, 430) keine gespeicherte Form haben und die Querstreben (450) verstreckt sind.

3. Implantierbare Vorrichtung nach einem der Ansprüche 1 bis 2, **dadurch gekennzeichnet, dass** die Vorformung der Vorformungs- und Versteifungsstruktur (410) im ersten Zustand des Materials so ausgebildet ist, dass der vorgeformte Abschnitt der Elektrodenleitung eine versteifte J-, eine versteifte Helix-, eine versteifte S-, eine versteifte Omega- oder eine versteifte gerade lang gestreckte Form aufweist.

4. Implantierbare Vorrichtung nach einem der Ansprüche 1 bis 3, **dadurch gekennzeichnet, dass** die Vorformung der Vorformungs- und Versteifungsstruktur (410) im zweiten Zustand des Materials so ausgebildet ist, dass der vorgeformte Abschnitt der Elektrodenleitung eine im Vergleich zum ersten Zustand formlose und elastische Form aufweist, insbesondere lang gestreckt und flexibel ist.

5. Implantierbare Vorrichtung nach einem der Ansprüche 1 bis 4, **dadurch gekennzeichnet, dass** das Material gegebenenfalls durch Energieeintrag und/oder Degradation zusätzlich in einen Zwischenzustand überführbar ist, der sich zwischen dem ersten und zweiten Zustand befindet, in dem es vorgeformt und/oder versteift ist, um die Vorformungs- und Versteifungsstruktur (410) in einer vom ersten Zustand unterschiedlichen Art und Weise vorzuformen und/oder zu versteifen.

6. Implantierbare Vorrichtung nach Anspruch 5, **dadurch gekennzeichnet, dass** die Vorformungs- und Versteifungsstruktur (410) im erster Zustand des Materials eine versteifte lang gestreckte Form, im Zwischenzustand des Materials eine versteifte J-, eine versteifte Helix-, eine versteifte S- oder eine versteifte Omega-Form und im zweiten Zustand des Materials eine im Vergleich zum ersten Zustand oder Zwischenzustand formlose und elastische Form aufweist, insbesondere lang gestreckt und flexibel ist.

7. Implantierbare Vorrichtung nach Anspruch 5 oder 6, **dadurch gekennzeichnet, dass** sich der Zwischenzustand des Materials innerhalb von 0,5 bis 10min und der zweite Zustand innerhalb von 1 bis 140 Tagen einstellt.

8. Implantierbare Vorrichtung nach einem der Ansprüche 5 bis 7, **dadurch gekennzeichnet, dass** sich der Zwischenzustand des Materials durch Energieeintrag, insbesondere Wärme im Bereich von 35°C bis 40°C, und/oder Degradation, und der zweite Zustand durch Degradation einstellt.

9. Implantierbare Elektrodenleitung nach einem der Ansprüche 1 bis 8, **dadurch gekennzeichnet, dass** das Material ein Shape-Memory Polymer und/oder ein biodegradierbares Polymer ist.

10. Implantierbare Vorrichtung nach Anspruch 9, **dadurch gekennzeichnet, dass** das Shape-Memory Polymer biodegradierbar ist.

## Claims

1. An implantable electrode lead, comprising:
- an elongated electrode lead body having a proximal and a distal end, at least one electrical supply line, which is surrounded by an electrically insulating material so as to electrically insulate the supply lines with respect to the surroundings of the electrode lead;
- at least one electrically active surface, which is located at or in the vicinity of the distal end and is connected to the at least one electrical supply line and which can emit therapeutic signals and/or pick up diagnostic signals;
- at least one connector, which is located at the proximal end and is electrically connected to the at least one electrical supply line and can be connected to an electromedical implant; and
- at least one preformed and/or reinforced section of the electrode lead body, which has an additional preforming and reinforcing structure (410), by way of which the section is preformed and/or reinforced, wherein the additional preforming and reinforcing structure comprises a material which is present in a first state, in which it is preformed and/or reinforced, and which, by way of energy application and/or degradation, can be transferred into a second state, in which the shape and/or the rigidity of the material, and thus of the preforming and reinforcing structure, is changed as compared to the first state;
**characterized in that**
the preforming and reinforcing structure (410) has a lattice-shaped design and surrounds the entire electrode lead body.

2. The implantable device according to claim 1, **characterized in that** the preforming and reinforcing structure (410) comprises first struts (420) having a first direction of winding and second struts (430) having a second direction of winding, wherein the first and second directions of winding are arranged in different directions around the electrode lead body so that the two strut sections intersect at intersecting points (440), wherein the first and second struts (420, 430) are elongated and have no inherent angles, the first and second struts (420, 430) are connected, preferably glued or welded, to each other at the intersecting points (440), the two struts (420, 430) cannot be displaced in relation to each other, the intersecting points (440) are additionally connected to each other by cross struts (450), and the first and second struts (420, 430) have no memorized shape and the cross struts (450) are drawn.

3. The implantable device according to either claim 1 or 2, **characterized in that**, in the first state of the material, the preformed shape of the preforming and reinforcing structure (410) is configured so that the preformed section of the electrode lead has a reinforced J, a reinforced helix, a reinforced S, a reinforced omega or a reinforced straight elongated shape.

4. An implantable device according to any one of claims 1 to 3, **characterized in that**, in the second state of the material, the preformed shape of the preforming and reinforcing structure (410) is configured so that the preformed section of the electrode lead has a formless and elastic shape as compared to the first state, in particular that it is elongated and flexible.

5. An implantable device according to any one of claims 1 to 4, **characterized in that** the material, optionally by way of energy application and/or degradation, can additionally be transferred into an intermediate state, which is between the first and second states and in which the material is preformed and/or reinforced so as to preform and/or reinforce the preforming and reinforcing structure (410) in a manner different from the first state.

6. The implantable device according to claim 5, **characterized in that** the preforming and reinforcing structure (410) in the first state of the material has a reinforced elongated shape, in the intermediate state of the material it has a reinforced J, a reinforced helix, a reinforced S or a reinforced omega shape, and in the second state of the material it has a formless and elastic shape as compared to the first state or the intermediate state, in particular it is elongated and flexible.

7. The implantable device according to claim 5 or 6, **characterized in that** the intermediate state of the material occurs within 0.5 to 10 minutes and the second state occurs within 1 to 140 days.

8. An implantable device according to any one of claims 5 to 7, **characterized in that** the intermediate state of the material occurs by way of energy application, in particular heat in the range of 35°C to 40°C, and/or degradation, and the second state occurs by way of degradation.

9. An implantable electrode lead according to any one of claims 1 to 8, **characterized in that** the material is a shape-memory polymer and/or a biodegradable polymer.

10. The implantable device according to claim 9, **characterized in that** the shape-memory polymer is biodegradable.

## Revendications

1. Ligne d'électrode implantable, comprenant :
- un corps de ligne d'électrode étiré en longueur, avec une extrémité proximale et une extrémité distale, au moins une ligne d'alimentation électrique entourée d'un matériau électriquement isolant pour isoler électriquement les lignes d'alimentation vis-à-vis de l'environnement de la ligne d'électrode,
- au moins une surface électriquement active située à l'extrémité distale ou à proximité de celle-ci et reliée à l'au moins une ligne d'alimentation électrique, par laquelle les signaux thérapeutiques peuvent être délivrés et/ou les signaux de diagnostic peuvent être reçus,
- au moins une prise mâle située à l'extrémité proximale, reliée à l'au moins une ligne d'alimentation électrique et apte à être reliée à un implant électro-médical, et
- au moins une section préformée et/ou renforcée du corps de ligne d'électrode, comportant une structure de préformage et de renforcement (410) supplémentaire permettant de préformer et/ou de renforcer la section, où la structure de préformage et de renforcement supplémentaire comprend un matériau présent dans un premier état, dans lequel il est préformé et/ou renforcé, et pouvant être mis dans un deuxième état par apport d'énergie et/ou dégradation, dans lequel la forme et/ou la rigidité du matériau et donc de la structure de préformage et de renforcement sont modifiées par rapport au premier état,
**caractérisé en ce que**
la structure de préformage et de renforcement (410) présente une construction du genre grille et encercle entièrement le corps de ligne d'électrode.

2. Dispositif implantable selon la revendication 1, **caractérisé en ce que** la structure de préformage et de renforcement (410) comporte des premières entretoises (420) avec un premier sens d'enroulement et des deuxièmes entretoises (430) avec un deuxième sens d'enroulement, dans lequel les premier et deuxième sens d'enroulement vont dans des direction différentes autour du corps de ligne d'électrode, de sorte que les deux entretoises se croisent en des points de croisement (440), dans lequel les premières et deuxièmes entretoises (420, 430) sont étendues en longueur et ne comportent pas d'angle en elles, les premières et deuxièmes entretoises (420, 430) sont reliées entre elles aux points de croisement (440), de préférence par collage ou soudage, les deux entretoises 420 et 430 ne sont pas déplaçables l'une par rapport à l'autre, les points de croisement (440) sont en outre reliés entre eux par des traverses (450), et les premières et deuxièmes entretoises (420, 430) ne présentent pas de forme enregistrée, et les traverses (450) sont étirées.

3. Dispositif implantable selon l'une des revendications 1 à 2, **caractérisé en ce que** le préformage de la structure de préformage et de renforcement (410) est conçu de telle manière dans le premier état du matériau, que la section déformée de la ligne d'électrode présente une forme en J renforcée, une forme hélicoïdale renforcée, une forme en S renforcée, une forme en oméga renforcée ou une forme droite étirée en longueur et renforcée.

4. Dispositif implantable selon l'une des revendications 1 à 3, **caractérisé en ce que** le préformage de la structure de préformage et de renforcement (410) est conçu de telle manière dans le deuxième état du matériau, que la section préformée de la ligne d'électrode présente une forme informe et élastique en comparaison avec le premier état, tout en étant en particulier étirée en longueur et souple.

5. Dispositif implantable selon l'une des revendications 1 à 4, **caractérisé en ce que** le matériau peut en outre être mis dans un état intermédiaire par apport d'énergie et/ou dégradation, lequel se situe entre le premier et le deuxième états, dans lequel le matériau est préformé et/ou renforcé pour préformer et/ou renforcer la structure de préformage et de renforcement (410) d'une manière différente du premier état.

6. Dispositif implantable selon la revendication 5, **caractérisé en ce que** la structure de préformage et de renforcement (410) présente une forme étirée en longueur dans le premier état du matériau, une forme en J renforcée, une forme hélicoïdale renforcée, une forme en S renforcée ou une forme en oméga renforcée dans l'état intermédiaire du matériau, et une forme informe et élastique, en particulier étirée en longueur et souple dans le deuxième état du matériau, en comparaison avec le premier état ou l'état intermédiaire.

7. Dispositif implantable selon la revendication 5 ou 6, **caractérisé en ce que** l'état intermédiaire du matériau est atteint en 0,5 à 10 minutes, et le deuxième état en 1 à 140 jours.

8. Dispositif implantable selon l'une des revendications 5 à 7, **caractérisé en ce que** l'état intermédiaire du matériau est atteint par apport d'énergie, en particulier une chaleur dans la plage de 35°C à 40°C, et/ou par dégradation, tandis que le deuxième état est atteint par dégradation.

9. Ligne d'électrode implantable selon l'une des revendications 1 à 8, **caractérisé en ce que** le matériau est un polymère à mémoire de forme et/ou un polymère biodégradable.

10. Dispositif implantable selon la revendication 9, **caractérisé en ce que** le polymère à mémoire de forme est biodégradable.
